# EUROPEAN PATENT SPECIFICATION

(11) **EP 1 797 884 B1**
(45) Date of publication and mention of the grant of the patent: **11.09.2013**
(21) Application number: 06019082.4
(22) Date of filing: 06.12.2000
(51) Int. Cl.: A61K 31/541, A61K 45/06, A61P 35/00

(54) **Taurolidine or taurultam for use in the treatment of tumors of the prostate, colon, lung and for the treatment of recurrent glioblastoma multiforme**
Taurolidin oder Taurultam zur Verwendung bei der Behandlung von Tumoren der Prostata, des Kolons, der Lungen und von wiederkehrendem Glioblastoma multiforme.
Taurolidine ou de taurultame pour i' utilisation dans le traitement des tumeurs de la prostate, des poumons, du côlon et dans le traitement de la glioblastome multiforme récurrente

(30) Priority: 06.12.1999 US 169122 P; 06.12.1999 US 169127 P; 06.12.1999 US 169128 P
(43) Date of publication of application: 20.06.2007
(62) Divisional of application: 00983965.5
(73) Proprietor: Geistlich Pharma AG, 6110 Wolhusen (CH)
(72) Inventor: Calabresi, Paul, deceased (US); Darnowski, James, Barrington, RI 02806 (US)
(74) Representative: Matthews, Derek Peter

(56) References cited:
- EP-A- 1 066 830
- WO-A-90/06138
- WO-A-92/00743
- WO-A-94/03174
- WO-A-98/52572
- WO-A1-95/18638
- WO-A2-01/39763

## Description

### BACKGROUND OF THE INVENTION

The invention relates to cancer therapy.

Despite advances in the identification of chemotherapeutic agents for inhibiting the growth of cancer cell, cancer remains a formidable disease with a high mortality rate. A significant problem of chemotherapeutic agents is low specificity. Many anticancer agents do not adequately distinguish normal cells from cancer cells. As a result, they often carry undesirable serious side effects.

### SUMMARY OF THE INVENTION

The invention provides a compound for use in inducing apoptosis in cancer cells in a mammal, wherein said compound is taurolidine or taurultam and wherein said cancer is selected from colon cancer, lung cancer and prostate cancer. The compound is administered to directly contact a tumor cell at a dose sufficient to induce cell death by apoptosis. Preferably the compound is administered in a manner and at a dose which preferentially induces apoptotic death compared to necrotic death. The compound is administered systemically, e.g., orally or intravenously. The compound to be administered is taurolidine or taurultam. Taurolidine is also provided for use in the treatment of advanced recurrent glioblastoma multiforme.

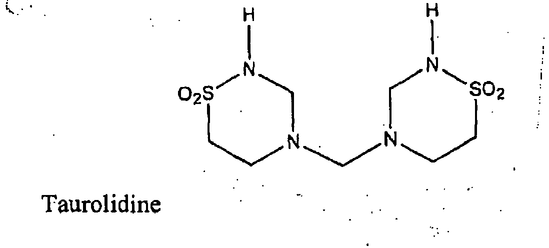

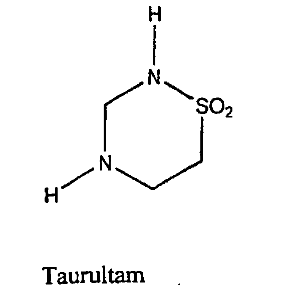

The compounds are administered alone or in combination with another antineoplastic agent. Preferably, the coadministered agent kills tumors cells by a mechanism other than apoptosis. For example, an antimetabolite, a purine or pyrimidine analogue, an alkylating agent, crosslinking agent (e.g., a platinum compound), and intercalating agent, and/or an antibiotic is administered in a combination therapy regimen. The coadministered drug is given before, after, or simultaneously with a taurolidine or taurultam .

The invention also includes treating a drug resistant tumor, e.g., a multiple drug resistant (MDR) tumor, in a mammal by administering to the mammal a taurolidine or taurultam compound. The tumor to be treated is a carcinoma . The drug resistant tumor is selected from the group consisting of colon cancer, prostate cancer, and lung cancer.

Preferably,taurolidine or taurultam is administered in a manner which allows direct contact of the surface of the tumor cell. The compound binds to a component, e.g., a cell surface polypeptide ligand or other cell surface moiety to initiate an intracellular signal transduction cascade culminating with cell death by apoptosis. Tumors to be treated colon cancer, prostate cancer and lung cancer. Slow release of the compound to any tissue is accomplished by implanting a drug loaded matrix in direct contact or adjacent to the tumor site.

The compounds are formulated for administration to directly contact cancer cells, e.g., in the form of an aqueous solution. Formulations include a therapeutic film-forming composition containing or coated with the therapeutic compound as well as ointments, pastes, sprays, patches, creams, gels, sponges, and foams.

Other features and advantages of the invention will be apparent from the following detailed description and from the claims.

### BRIEF DESCRIPTION OF THE DRAWINGS

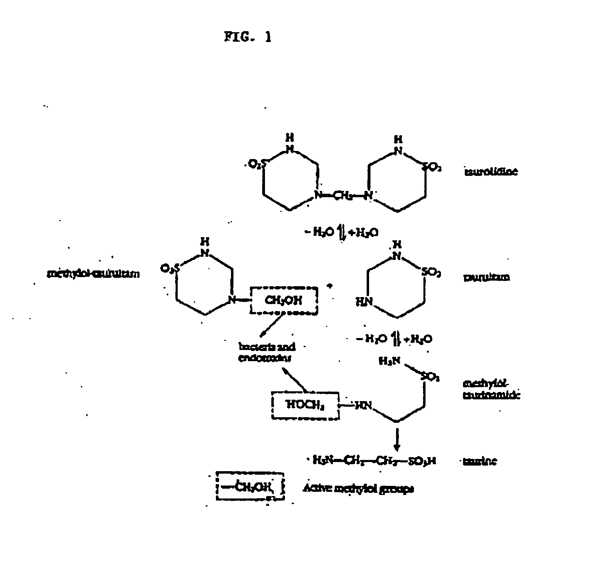

Fig. 1 is a diagram of the structure of Taurolidine and its major breakdown products or metabolites (taurultam, taurinamide and taurine). Upon breakdown, each molecule of Taurolidine generates 3 methylol containing moieties implicated the antibiotic and anti-endotoxin activities of taurolidine.

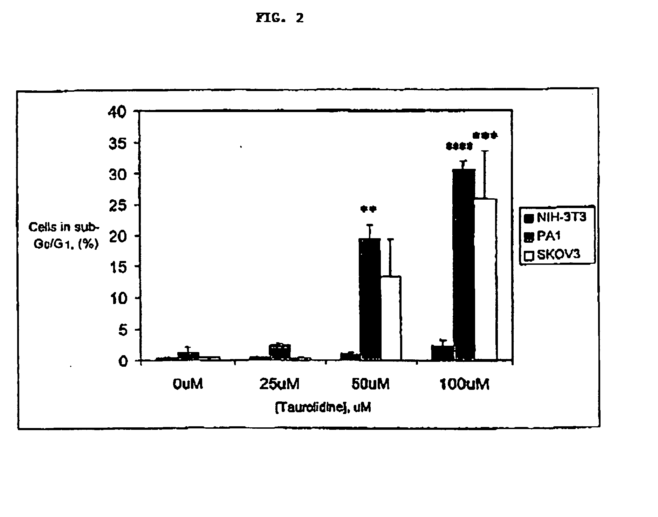

Fig. 2 is a bar graph showing the effect of a 48h exposure to Taurolidine on the appearance of DNA debris in PA-1. SKOV-3 and NIH-3T3 cells. Three x 10⁵ cells were seeded in plastic tissue culture flasks, Twenty-four hours later. Taurolidine was added to achieve final concentrations of 25 µM. 50 µM or 100 µM. Control cultures received an appropriate volume of Kollidine-17P. After a 48h period of Taurolidine exposure, cells were harvested and stained with propidium iodide. The percentage of DNA debris in the sub-G₀/G₁ region was assessed using cytofluorometric techniques. Each bar represents the mean (±SE) of three determinations. ** *p*≤0.01. *** *p*≤0.001, **** *p*≤0.0001

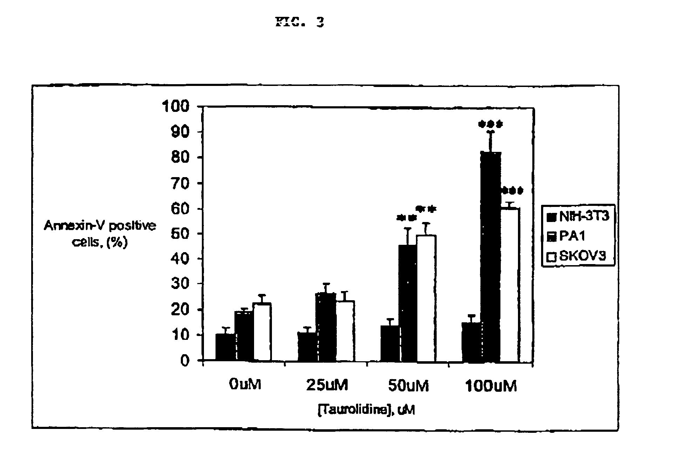

Fig. 3 is a bar graph showing the effect of a 24h exposure to Taurolidine on membrane phosphotidylserine externalization in PA-1. SKOV-3 and NIH-3T3 cells. Three x 10⁵ cells were seeded in plastic tissue culture flasks. Twenty-four hours later, Taurolidine was added to achieve final concentrations of 25 µM. 50 µM or 100 µM. Control cultures received an appropriate volume of Kollidine-17P. After an additional 24h. cells were harvested and phosphotidylserine externalization determined by assessing Annexin-V FITC binding using cytofluorometric techniques. Each bar represents the mean±SEM of four determinations.** *p*≤0.01. *** *p*≤0.001

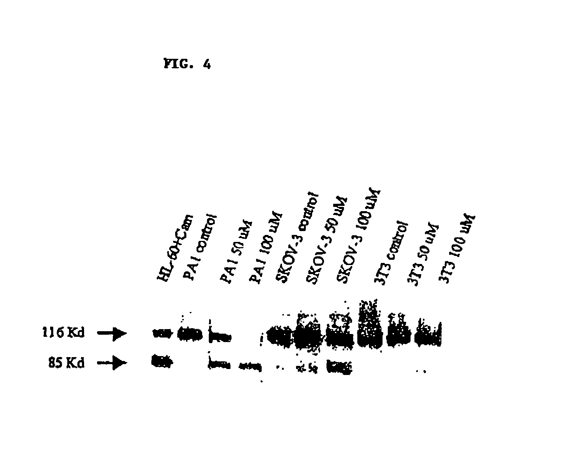

Fig. 4 is a photograph of showing the results of a Western-blot analysis of the effect of a 24h exposure to 50 or 100 µM Taurolidine on PARP expression and the appearance of a major PARP cleavage product in PA-1, SKOV-3 and NIH-3T3 cells. Two x 10⁶ cells were seeded in 150 cm² tissue culture flasks. Twenty-four hours later, Taurolidine was added at concentrations of 50 µM or 100 µM. After an additional twenty-four hours, cells were harvested, cell number determined, and aliquots derived from equal cell numbers generated from each exposure condition. Total proteins from these whole cell lysates were separated by SDS-PAGE and transferred to nitrocellulose filters. Filters were then immunoblotted to detect intact PARP protein and cleavage fragments by using the clone C-2-10 mouse monoclonal anti-PARP antibody (Zymed Laboratories. San Francisco, CA). The resulting protein-antibody complexes were visualized by chemiluminescence techniques.

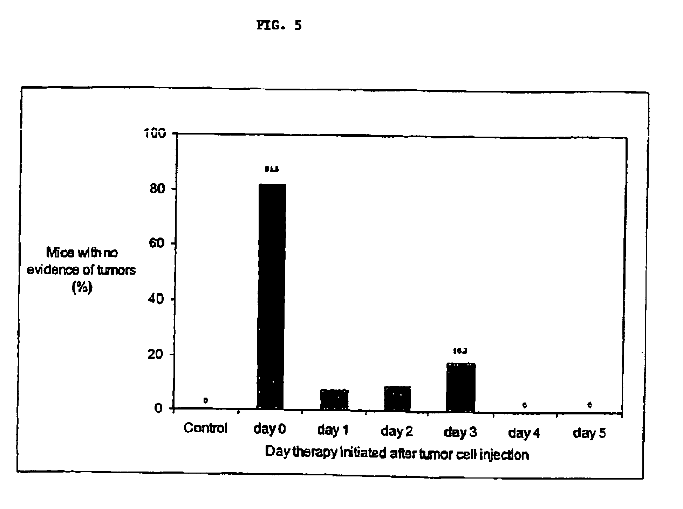

Fig. 5 is a bar graph showing the effect of delayed administration of a single 3-day (3d) intraperitoneal (i.p.) bolus injection regimen of Taurolidine (20 mg/mouse/injection) on the occurrence of i.p. human tumor xenografts in female nude mice following the i.p. administration of 5 x 10⁶ SKOV-3 human ovarian turnor cells. Taurolidine therapy was initiated on the day of tumor cell inoculation or up to 5d thereafter. Fourteen days following the final Taurolidine injection, mice in all groups were sacrificed and the peritoneal cavity examined for the presence of tumors. Each experimental was repeated three times and the pooled number of animals in each group ranged from 15-21.

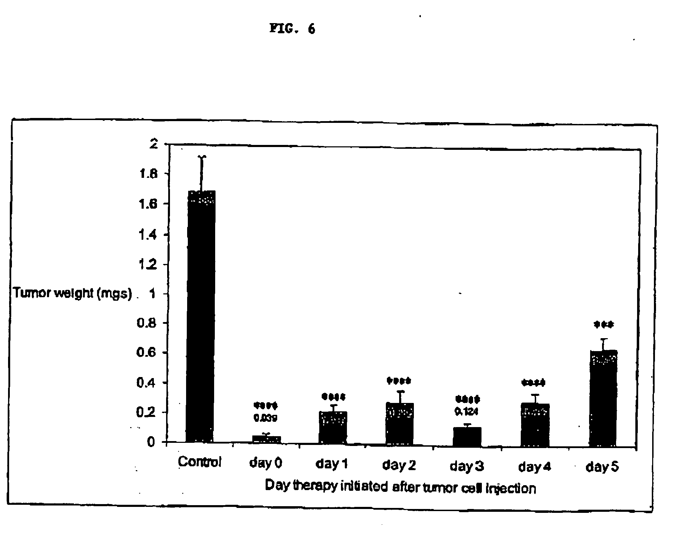

Fig. 6 is a bar graph showing the effect of delayed administration of a single 3d i.p. bolus injection regimen of Taurolidine (20 mg/mouse/injection) on the weight of i.p. human tumor xenografts in female nude mice following the i.p. administration of 5 x 10⁶ SKOV-3 human ovarian tumor cells. Taurolidine therapy was initiated on the day of tumor cell inoculation or up to 5d thereafter. Fourteen days following the final Taurolidine injection, mice in all groups were sacrificed. i.p. ovarian tumor xenografts removed and tumor weighed. Each experiment was repeated three times and the pooled number of animals in each group ranged from 15-21. Each bar represents the mean (±SE) tumor weight of 15-21 animals. *** *p*≤0.001, **** *p*≤0.0001.

### DETAILED DESCRIPTION

Taurolidine and taurultam were found to be safe and effective antineoplastic agents which preferentially induce apoptotic death in cancer cells. The compounds induce apoptotic death of tumor cells.

### Therapeutic Compounds

Taurolidine was found to be selectively toxic to cancer cells without killing normal (i.e., noncancerous) cells. Taurolidine, taurultam, or derivatives or metabolites thereof have high affinity for and binds selectively to a moiety on the surface of a cancer cell (e.g., a phosphatidyl serine receptor) and induce apoptosis in that cell, which in turn leads to cytotoxicity. Cellular internalization of the compound may not be necessary for induction of apoptotic death of a cancer cell.

Cytotoxicity or cell death may occur by either necrosis or apoptosis. Necrosis, which is not genetically controlled, is usually the result of physical or chemical injury. Apoptosis is genetically controlled and is a cellular response to a specific stimuli, e.g., a cell surface-generated signal. Necrosis involves the destruction of cytoplasmic organelles and a loss of plasma membrane integrity, whereas cells undergoing apoptosis exhibit cell shrinkage, membrane blebbing, chromatin condensation and fragmentation. After the DNA damage in the caspase enzyme pathway, there are a series of events which occur that involve calcium activation and calpain enzymes which further leads to other cellular changes and regulation of cytoplasmic enzymes.

A major difference between necrosis and apoptosis *in vivo* is the elimination of the apoptotic cell before an inflammatory response is mounted. In contrast to apoptosis of cells, necrosis of cells causes inflammation. Thus, induction of cytotoxicity of cancer cells by apoptosis offers considerable advantages over induction of cell death by convention chemotherapeutic agents because apoptotic death is associated with minimal damage to surrounding cells or tissue. Unlike many conventional chemotherapeutic agents, taurolidine or taurultam compounds administered according to the invention are cytotoxic agents which induce apoptosis of cancer cells (but not normal noncancerous cells) to safely reduce the tumor burden in a mammal suffering from cancer.

### Functional characterization of Taurolidine

Taurolidine (Taurolin^{™}) is chemically identified as Bis-(1,1-dioxoperhydro-1,2,4-thiadiazinyl-4)methane (Fig. 1). It is a relatively small dimeric molecule with a molecular weight of 284 (Knight et al., 1983. J. Pharm. Sci 72:705-707)). Early assessment of its antibiotic activity revealed that it possessed bactericidal activity against a broad spectrum of aerobic and anaerobic bacterial strains, The minimum concentration required to inhibit bacterial cell growth (MIC) ranged from 0,01 to 1mg/ml, depending on the bacterial strain evaluated. Early studies also revealed that Taurolidine possessed activity against clinically relevant fungi. The concentration required to exert antifungal activity is approximately equivalent to that required to produce its antibacterial activity.

The antibiotic activity of Taurolidine depends upon a chemical reaction secondary to the generation of active methylol groups formed upon the decomposition of the parent Taurolidine molecule (Fig. 1). Biochemical and morphological studies revealed that Taurolidine-derived methylol group containing moieties appeared to react with bacterial cell wall components. The result of this chemical reaction is that exposure to this agent significantly inhibits the ability of microorganisms to adhere to biological surfaces, such as epithelial cells. Exposure to Taurolidine disrupted the structure, and reduced the number, of bacterial cell fimbriae, apparently a reflection of the agglutination of these structures. Modification of these surface structures is thought to be responsible for the ability of Taurolidine to disrupt bacterial cell adhesion. In addition to this direct effect on bacterial cell wall components. Taurolidine also possesses anti-endotoxin activity by reducing tumor necrosis factor alpha (TNF-α) synthesis and activity. Taurolidine also reduces the extent and severity of postoperative peritoneal adhesions and has been administered clinically, by lavage, after abdominal surgery to reduce post-operative infections and adhesions as well as to treat peritonitis.

Taurolidine is a synthetic broad-spectrum antibiotic that also possesses antifungal activity. Mechanistically, it reacts with bacterial cell membrane components to prevent the adhesion of bacterial cells to epithelial cell surfaces. Reflecting the key role of cell adhesion in the growth and development of human solid tumors, studies were initiated to assess the cytotoxic activity of this agent against the growth of a panel consisting of 12 selected human and murine tumor cell lines. Assessment of the growth inhibitory activity of a 3d Taurolidine exposure revealed that this agent inhibited the growth of all cell lines evaluated with IC₅₀s ranging from 9.6-34,2 µM. Studies to identify the underlying mechanism(s) responsible for this effect were conducted in NIH-3T3 murine fibroblasts and the PA-1 and SKOV-3 human ovarian tumor cell lines. Initial studies assessed the effect of a 48h exposure to Taurolidine on cell cycle distribution. The results of this analysis revealed that while Taurolidine had little effect on the cell cycle in PA-1 cells, in SKOV-3 cells it reduced the percentage of cells in the G₀/G₁-phase and increased the percentage of cells in both S and G₂/M. In these human tumor cell lines. Taurolidine exposure significantly increased DNA debris in the sub-G₀/G₁ region, an effect consistent with an induction of apoptosis. In contrast, in NIH-3T3 cells. Taurolidine increased the percentage of cells in S-phase, decreased the percentage of cells in G₀/G₁, and did not increase DNA debris in sub-G₀/G₁ region. Further studies of the relationship between Taurolidine exposure and tumor cell apoptosis assessed phosphotidylserine externalization following a 24h exposure to Taurolidine, using Annexin-V binding as a cell surface marker. These studies revealed that Taurolidine increased the percentage of Annexin-V positive cells by 4- and 3-fold in PA-1 and SKOV-3 cells, respectively. In contrast, in NIH-3T3 cells. Taurolidine exposure slightly increased (~5%) Annexin-V binding, Complementary studies determined if a 48h exposure to either 50 or 100 µM Taurolidine affected PARP cleavage in these cell models and revealed that Taurolidine induced PARP cleavage in both PA-1 and SKOV-3 cells. In total, these *in vitro* results reveal that Taurolidine possesses tumor cell cytotoxic activity that correlates with its ability to specifically induce apoptosis. Finally, murine-based studies were conducted to assess the antineoplastic activity of this agent. Initial studies assessed the toxicity of 3 consecutive daily i.p. bolus injections of Taurolidine, at doses ranging from 5mg/inj/mouse-30mg/inj/mouse. The 20mg/inj dose produced ~10% mortality and was identified as the MTD in this model. Administration of this Taurolidine regimen to nude mice bearing i.p. human ovarian tumor xenografts resulted in a significant inhibition of both tumor formation and growth. These findings reveal that Taurolidine may represent a novel class of antineoplastic agent and are discussed in light of their clinical implications.

The invention is based on the discovery that, in addition to the activities discussed above, taurolidine selectively and reliably inhibits tumor cell growth and selectively kills tumor cells by inducing apoptosis. Taurolidine has now been found to kill at least 28 different human tumor cell lines including ovarian, breast, brain, colon, prostate, urinary bladder and lung tumors, as well as melanomas, mesotheliomas, laryngeal carcinomas, leukemias, and lymphomas. In addition, multiple-drug resistant glioma clls and myelodysplastic syndrome cells (a precancerous cell type) were killed by taurolidine. Inhibition of tumor growth and induction of apoptotic tumor cell death occur at taurolidine concentrations significantly lower than those required for antibiotic activity. For example, for antineoplastic applications, taurolidine is administered at a dose that is at least 10% less, preferably at least 20% less, more preferably at least 50% less, and up to one log unit less than the dose required for antibacterial or antiadhesive activity.

Taurolidine is toxic to tumor cells (but not normal non-tumor cells) regardless of the tumor origin. Apoptosis of tumor cells s induced after an incubation with Taurolidine for as little as one hour in culture.

Taurolidine and metabolites thereof are also useful in combination therapy. The data indicate that taurolidine is useful to enhance the cytotoxicity of other chemotherapeutic agents and/or radiation therapy by inducing certain types of cancer cells to enter "S" phase.

### Taurolidine and Angiogenesis

Patients with metastatic colon cancer were treated with taurolidine and several factors controlling angiogenesis were measured. Four factors controlling the growth of blood vessels (tissue necrosis factor (TNF); interleukins 1.6, and 10; vascular endothelial growth factor (VEGF); and tumor growth factor-β (TGF)) were found to be decreased in taurolidine-treated subjects compared to subjects receiving placebo. These data indicate that taurolidine as an anti-angiogenesis agent.

### Therapeutic Administration

An effective amount of a therapeutic compound is preferably from about 0.1 mg/kg to about 150 mg/kg. However, due to the low toxicity of taurolidine and taurultam compounds, higher doses may be administered without deleterious side effects. A dose effective to induce apoptosis of cancer cells is an order of magnitude less than doses administered for antiseptic, antibacterial. antitoxic, or anti-adhesion purposes. An apoptotic dose of taurolidine or taurultam effective to induce apoptosis (e.g., 0.5 µg/dl) is also significantly less than doses previously suggested (e.g., 150-450 mg/kg) as potentially being useful in the treatment of certain cancers. Effective doses vary, as recognized by those skilled in the art, depending on route of administration. excipient usage, and coadministration with other therapeutic treatments including use of other antitumor agents (e.g., an antimetabolite, a purine or pyrimidine analogue, an alkylating agent crosslinking agent, intercalating agent, or an antibiotic.) and radiation therapy.

A therapeutic regimen is carried out by identifying a mammal, e.g.. a human patient suffering from (or at risk of developing) colon cancer, lung cancer or prostate cancer using standard methods. For example, taurolidine or taurultam is administered to an individual diagnosed with a cancer or an individual diagnosed with a precancerous condition The pharmaceutical compound is to administered to such an individual using methods known in the art. Preferably, the compound is administered orally, topically or parenterally, e.g., subcutaneously, intraperitoneally, intramuscularly, and intravenously. The compound is administered prophylactically, after the detection of a recurring tumor, or at the time of surgery. Examples of formulations suitable for parenteral administration include aqueous solutions of the active agent in an isotonic saline solution, a 5% glucose solution, or another standard pharmaceutically acceptable excipient. Standard solubilizing agents such as PVP or cyclodextrins are also utilized as pharmaceutical excipients for delivery of the therapeutic compounds.

The therapeutic compounds described herein are formulated into compositions for other routes of administration utilizing conventional methods. For example, taurolidine or taurultam can be formulated in a capsule or a tablet for oral administration. Capsules may contain any standard pharmaceutically acceptable materials such as gelatin or cellulose. Tablets may be formulated in accordance with conventional procedures by compressing mixtures of a therapeutic compound with a solid carrier and a lubricant. Examples of solid carriers include starch and sugar bentonite. The compound is administered in the form of a hard shell tablet or a capsule containing a binder, e.g., lactose or mannitol, a conventional filler, and a tableting agent. Other formulations include an ointment, paste, spray, patch, cream, gel, resorbable sponge, or foam. Such formulations are produced using methods well known in the art.

Taurolidine or taurultam compounds are effective upon direct contact of the compound with the cancer cell. Accordingly, the compound may be administered topically.

Alternatively, the compounds are administered by implanting (either directly into an organ such as the liver or subcutaneously) a solid or resorbable matrix which slowly releases the compound into adjacent and surrounding tissues of the subject.

For treatment of glioblastomas, the compound is systemically administered. The compound is for example administered intravenously.

### Cytotoxicity of taurolidine and taurultam compounds

The cytotoxic activity of taurolidine was evaluated *in vitro* against the growth of a variety of human cancer cell lines as well as "normal" NIH 3T3 fibroblasts and found to induce apoptotic cytotoxicity. The neoplastic cell lines used in the survey were standard tumor cell lines, e.g., PA1 human ovarian cell line, SKOV3 human ovarian cell line, HT29 human colon tumor cell line, DU145 human prostate tumor cell line, U251 human glioblastoma cell line, U251-MDR human glioblastoma cell line transfected with DNA encoding MDR, T98G human glioblastoma cell line, SP-1 human leukemia cell line, and Daudi human leukemia cell line.

The data indicated that taurolidine inhibited human cancer cell growth. Surprisingly, the concentration of taurolidine required to inhibit tumor cell growth after a 3-day exposure to the compound (IC₅₀) was approximately 12.5 µM - 50 µM. This concentration is at least 1000-fold lower than concentrations used to inhibit bacterial cell growth.

Taurolidine and cancer cells were added to flasks simultaneously, and cell growth was assessed 3 days later. Parallel studies were carried out to assess whether disruption of cell adhesion played a role in the cytotoxic activity. Assays were carried out to assess the ability of taurolidine to inhibit the growth of human ovarian tumor cells after they were established and growing *in vitro* as discrete colonies. The data revealed that a 24-hour exposure to 50 µM taurolidine produced a significant cytotoxic effect against the growth of established tumor cells. The data indicated that the cytotoxic/cytostatic activity of taurolidine is not due to inhibition of tumor cell adhesion.

The mechanism by which taurolidine produces cytoxicity was evaluated. Cell cycle kinetics and cell cycle distribution of tumor cells were examined after a 24-hour exposure to taurolidine. The results revealed that in both PA1 and 3T3 cells, taurolidine exposure disrupted cell cycle kinetics and significantly reduced the percentage of cells in both the Sand G2M-phases. Exposure of PA1 human ovarian cells to this regimen of taurolidine also induced a high degree of DNA fragmentation indicating the induction of apoptosis. This DNA fragmentation was not observed in normal 3T3 cells.

To further evaluate the possibility that exposure to 50 µM taurolidine was capable of specifically inducing apoptosis in human ovarian tumor cells but not normal fibroblasts, studies were undertaken to evaluate DNA fragmentation as a function of taurolidine exposure by using agarose-gel electrophoresis. The results confirmed that, in ovarian tumor cells, exposure to taurolidine resulted in overt DNA fragmentation which was not apparent in 3T3 cells exposed to an identical taurolidine regimen.

The cytotoxic activity of taurultam was evaluated *in vitro* using the same human cancer cell lines as described above. The data indicated that taurultam induced apoptotic death of cancer cells but not normal control cells in the same manner as taurolidine. The cytotoxic activity of taurultam was approximately 75% of the activity observed with taurolidine.

Apoptotic death is distinguished from death by other mechanisms using methods known in the art. Another early reflection of the induction of apoptosis is the cleavage of the protein poly (ADP-ribose) polymerase (PARP) by cellular caspases. Western-blot based studies were carried out to determine if exposure to taurolidine resulted in PARP cleavage. The results revealed that PARP cleavage was not evident in 3T3 cells when exposure to the same taurolidine regimen. Apoptosis is also detected using known methods such as determination of caspase activation, bax/bcl12 ratios and fas and fas-I interactions. Other methods of distinguishing between apoptosis and necrosis (e.g.. a fluorescence-based method described in U.S. Patent No. 5. 976,822) are used to determine the mechanism of death or the dose at which a compound induces apoptosis compared to necrosis.

The antitumor activity of a compound is also evaluated using a standard MTS colorimetric assay. Results obtained with various types of tumor cells (primary cells or cell lines) are compared with those obtained by using normal cells. Viability of the cells in each cell line is estimated by measuring the cellular conversion of a tetrazolium salt after incubating the cells in a solution containing a test compound in a 96 well plate. IC₅₀ values obtained using the identical test compound on normal cells and cells of a particular tumor cell line are compared and their ratio (IC₅₀ normal cell/IC₅₀ cancer cell) indicates the cancer selectivity of the test compound. An increase in the IC₅₀ normal cell/IC₅₀ cancer cell ratio reflects a higher selectivity of the test compound to kill the cancer cell.

Antitumor activity of a compound is also evaluated *in vivo* using. e.g., a tumor xenograft regression assay. For example, animals bearing established tumors are treated with a test compound for a three-week period. The growth of the tumors and the general health of the animal are monitored during the three-week treatment and for two more weeks after treatment to determine if tumor regrowth occurs. The antineoplastic activity of taurolidine is determined in athymic (nude) mice bearing advanced and/or metastatic xenografts. Single and multiple dose regimens of taurolidine are evaluated in athymic (nude) mice. Upon identification of dose regiments, antineoplastic activity is assessed in athymic (nude) mice bearing xenografts of human cancer cells, e.g., ovarian, prostate, colon, pancreatic, breast and glioma tumors.

### Treatment of Drug Resistant tumors

Taurolidine was found to be particularly effective in killing tumor cells which are refractory to cytotoxicity by other known chemotherapeutic agents. Glioblastoma cells were transfected with a gene encoding multiple drug resistance (MDR). The transfected cells were 100-1000 times resistant to standard chemotherapeutic agents. e.g., adriamycin.

Untransfected glioblastoma cells cultured with a standard dose (e.g., 1µM) of adriamycin were killed, but MDR-transfected glioblastoma cells contacted with 1µM of the drug were resistant. Significant cytotoxicity of the MDR-transfected glioblastoma cells was observed after contact with the compound (e.g., taurolidine at a dose of 50µM). These data indicate that the compound described herein exert their cytotoxic activity via a mechanism that differs from that of standard chemotherapeutic agents. Accordingly, combination therapy in which a taurolidine or taurultam compound is administered before, after, or together with another chemotherapeutic agent (e.g., an antimetabolite, a tumor-specific monoclonal antibody, or anti-angiogenic agent) results in improved clinical outcome of patients suffering from a malignant condition characterized by a mixed population of tumor cells (e.g., those which are killed by standard chemotherapeutic agents and those which are MDR).

The following examples demonstrate apoptotic activity on a variety of cell lines, including prostate, colon and lung cancer cells (according to the invention) and ovarian cancer cells (according to EP-B-1 246 617, the patent granted on the parent application). In addition, a clinical trial against recurrent glioblastoma multiforme is reported.

WO 92/00743 and WO 98/52572 disclose the use of taurolidine taurultam for the treatment of lymphomas, sarcomas, melanomas, carcinomas and leukemias.

### Example 1: Cytotoxic and mechanistic evaluation of antineoplastic agents

Taurolidine was found to be active at inhibiting the growth of a variety of human tumor cell lines *in vitro.* PA-1 and SKOV-3 human ovarian tumor cell lines and NIH-3T3 murine fibroblasts were used to determine the mechanism of antitumor activity. The studies revealed that this effect was associated with alterations in DNA structure, cell membrane components, and protein cleavage that were consistent with the induction of apoptosis specifically in tumor cells. Antineoplastic evaluation of Taurolidine in nude mice bearing intraperitoneal xenografts of human ovarian tumors demonstrated that this agent significantly inhibited tumor development and growth *in vivo.*

To study neoplastic activity, Taurolidine was formulated as 2% solution in 5% Kollidon 17PF. Standard cell culture growth media (e.g., High glucose DMEM. RPMI 1640. McCoy's 5A, and F12K), trypsin, and fetal bovine serum (FBS) were all purchased from GIBCO/Life Technologies (Grand Island, NY). Phosphotidylserine externalization by cells was evaluated using the ApoAlert® Annexin-V/FITC assay kit was purchased from Clontech (Palo Alto, CA). Reagents for SDS-PAGE were purchased from BioRad Laboratories (Richmond, CA). A murine monoclonal antibody (clone C-2-10) to human PARP was purchased from Zymed Laboratories (San Francisco. CA). All other chemicals were purchased from Sigma (St. Louis. MO).

Studies to assess the cytotoxic activity of Taurolidine were carried out using a panel of human solid tumor cell lines as well as in NIH-3T3 murine fibroblasts. Included in the tumor cell line panel were ovarian tumor cells (PA-1 and SKOV-3), colon tumor cells (HCT-8, HCT-15 and HT-29), lung tumor cells (H-157, A-549 and H-596), prostate tumor cells (DU-145), glioma cells (U-251), and melanoma (MNT-1). The murine melanoma B16F10 cell line was also tested. These cell lines readily available, e.g., from the American Type Culture Collection (ATCC). Cells were cultured in appropriate growth medium at 37°C in a humidified incubator in an atmosphere of 5% CO₂. Under these growth conditions, the doubling time of all cell lines was 20-28h.

Studies to assess *in vivo* toxicity and therapeutic effectiveness were carried out in 6-12 week old female homozygous athymic (Hsd:athymic nude nu/nu) mice obtained from Harlan (Indianapolis. IN).

To evaluate inhibition of cell growth, subconfluent cultures of appropriate cell lines were harvested by trypsinization and resuspended in media at a cell density of 1-5x10⁴ cells/ml. One ml of this cell suspension was added to each well of a 12 well cell culture plate that contained 3ml of appropriate media plus serum. Twenty-four hours later. Taurolidine was added to each well, in a volume of 40 µl, to achieve a final concentration of 0.1-200 µM. Control wells received 40 µl of 5% Kollidon 17PF alone. Seventy-two hours later, all cells were harvested by trypsinization and cell number determined electronically using a Coulter Model Z1 particle counter (Coulter Corp.. Miami. FL) to assess cell growth inhibition. Each experiment was performed in duplicate and repeated a minimum of three times.

For flow cytometry studies. 1 x10⁶ PA-1. SKOV-3, or NIH-3T3 cells were incubated for 24h in appropriate media containing serum. Twenty-four hours later. Taurolidine was added in a volume of 40 µl to achieve a final concentration of 25. 50. or 100 µM. Control cultures for each cell line were incubated in media containing 40 µl of 5% Kollidon 17PF alone. Forty-eight hours later, all cells were harvested by trypsinization and prepared for cytofluorometric analysis by standard methods. For example, harvested cells were resuspended in ice cold phosphate-buffered saline at a final cell density of 2 x 10⁶ cells/ml. The cells then were stained for 30min at room temperature in the dark with a solution of 0.05 mg/ml propidium iodide. 0.6% Igepal, and 1% sodium citrate. Flow cytometry was performed by FACScan (Becton Dickinson, Plymouth, England) using the ModFit LT program (Becton Dickinson). Statistical analysis was performed with the Kruskal Wallis non-parametric ANOVA test followed by Dunn's multiple comparisons test using Instat.

Cell membrane phosphotidylserine externalization, as a reflection of the potential induction of apoptosis, was assessed by flow cytometry methods using the ApoAlert® Annexin-V/FITC assay kit. Briefly, 1 x 10⁶ cells were incubated for 24h in tissue culture medium containing serum. Thereafter. Taurolidine was added to achieve a final concentration of 25, 50, or 100 µM. Control cultures received 5% Kollidon 17PF alone. Twenty-four hours later, aii ceiis were harvested by trypsinization. The harvested cells were resuspended in 200 µl of binding buffer and then incubated for 5-15min in a solution containing 1 µg/ml Annexin-V FITC at room temperature in the dark. The cells were then analyzed to quantitate Annexin-V binding by cytofluorometric techniques that utilized FACScan using the ModFit LT program with statistical analysis as described above.

Western-blot analysis was used to assess of PARP cleavage. Two x 10⁶ cells were seeded into separate 75cm² tissue culture flasks containing 20 ml of tissue culture media plus serum. Twenty-four hours later, Taurolidine was added at concentrations of 50 µM or 100 µM. Twenty-four hours after the addition of Taurolidine, cells were harvested, cell number determined, and aliquots containing an equal cell number were generated from each exposure condition. Total proteins from whole cell lysates generated from these aliquots were separated by SDS-PAGE and electrotransferred to nitrocellulose filters. Filters were then processed to detect intact PARP protein and cleavage fragments by using the clone C-2-10 mouse monoclonal anti-PARP antibody (Zymed Laboratories. San Francisco. CA). The resulting protein-antibody complexes were visualized by standard chemiluminescence techniques.

To evaluate Taurolidine-induced toxicity, mice were divided into groups of 5-8 animals. Thereafter, all mice were weighed and therapy, consisting of a single i.p. bolus injection of Taurolidine on 3 consecutive days, was initiated. The Taurolidine doses evaluated were 5. 10, 15.20.25, and 30 mg/mouse/injection and, except for the 25 (1.25ml) and 30 mg/mouse (1.5ml) injections, were administered in a volume of 1ml. Taurolidine for injection was diluted from the 2% Taurolidine solution by the addition of 5% Kollidon 17PF. Control animals received I ml injections of 5% Kollidon 17PF alone. Animals were examined daily and body weight recorded twice weekly. A reduction in body weight of greater than 10% was considered significant. The maximally tolerated dose (MTD) was considered to be the dose which produced ~10% mortality.

To evaluate therapeutic effectiveness, mice received a single intraperitoneal injection of 5 x 10⁶ SKOV-3 cells in a volume of 0.5ml. Immediately thereafter, mice were randomly divided into treatment groups of 7 animals. Taurolidine therapy, consisting of a single i.p. bolus injection of 20 mg of Taurolidine on 3 consecutive days, was initiated either immediately following tumor cell inoculation or at selected time intervals after tumor cell inoculation (≤5d). Control animals received 1ml injections of 5% Kollidon 17PF alone. Animals were examined daily and body weight recorded twice weekly. Fourteen days following the last Taurolidine injection, mice in all groups were sacrificed by CO₂ asphyxiation, all i.p. tumor foci removed and tumor weighed determined. The mean tumor weight for each treatment group was calculated and statistical analysis of differences in the mean tumor weight between treatment groups employed the Student's *t*-test. *p*-values of <0.05 were considered significant.

### Taurolidine inhibits tumor cell growth

The ability of Taurolidine to inhibit cell growth was assessed in a panel of human and murine neoplastic cell lines comprised of 13 different lines representing 6 different tumor types. The results of this survey revealed that a 3d exposure to Taurolidine inhibited cell growth in each cell line examined (Table 5).

The IC₅₀ of Taurolidine against the growth a selected human and murine neoplastic cell lines was evaluated as follows. Cells were seeded at a density of 1-5 x 10⁴ cells in each well of a 6 well tissue culture flask. Twenty-four hours later. Taurolidine was added at concentrations ranging from 1-100 µM. After three days, cells were harvested by trypsinization and cell number determined electronically. Cell growth inhibition was determined by comparison to non-Taurolidine exposed control cultures. The IC₅₀ was calculated as the concentration required to inhibit cell number by 50%. Each IC₅₀ value represents the mean±SE of 4-8 determinations.

**Table 5**

| Tumor site of origin | Cell line | IC₅₀ (µM) |
|---|---|---|
| Ovary | PA-1 | 11.4±1.8 |
| | SKOV-3 | 31.6±7.0 |
| | | |
| Prostate | DU-145 | 9.8±0.8 |
| | | |
| Brain | U-251 | 20.1±2.7 |
| | | |
| Colon | HT-29 | 18.6±1.0 |
| | HCT-8 | 11.5±0.5 |
| | HCT-15 | 9.6±3.0 |
| | | |
| Melanoma | B16-F10 | 30.1±2.6 |
| | MNT-1 | 22.1±2.1 |
| | | |
| Lung | H-157 | 32.2±5.6 |
| | A-549 | 26.8±7.2 |
| | H-596 | 34.2 |
| | | |
| Murine fibroblasts | NIH-3T3 | 11.9±1.8 |

Surprisingly, the observed IC₅₀s for each cell line were remarkably similar and varied over the relatively narrow range of ~10 µM (PA-1, DU-145, HCT-8, HCT-15, B16F10, and NIH-3T3) to ~35 µM (H-596).

The studies assessed the effect of Taurolidine on tumor cell proliferation. Inhibition of proliferation could reflect either growth arrest or cell death. Therefore, studies were next focused to identify the mechanism(s) by which Taurolidine induced cell growth inhibition. These studies were carried out in the human ovarian tumor cell lines PA-1 and SKOV-3 and in NIH-3T3 murine fibroblasts. Studies employing conventional flow cytometry techniques assessed the effect of a 48h exposure to Taurolidine on cell cycle distribution in both the PA-1 and SKOV-3 human ovarian tumor cell lines. The results of these studies revealed that exposure to this agent did not induce a consistent pattern of cell cycle alterations.

The effect of a 48h exposure to selected concentrations of Taurolidine on cell cycle distribution in human ovarian tumor cells (PA-1 and SKOV-3) and murine fibroblasts (NIH-3T3) was carried out as follows. Three x 10⁵ cells were seeded in plastic tissue culture flasks. Twenty-four hours later, Taurolidine was added to achieve final concentrations of 25 µM, 50 µM or 100 µM. Control cultures received an appropriate volume of Kollidine-17P. After an additional 48h, cells were harvested, stained with propidium iodide, and cell cycle distribution assessed using cytofluorometric techniques. Each value represents the percentage of cells in the noted cell cycle phases and is the mean±SEM of three determinations.

**Table 6**

| Cell Line/Drug Exposure | Cell Cycle Distribution, (%) | | |
|---|---|---|---|
| | G₀G₁ | S | G₂/M |
| | | | |
| NIH-3T3 | | | |
| 48h-0µM Taurolidine | 46.1±9.2 | 45.0±5.9 | 9.0±3.3 |
| 48h-25µM Taurolidine | 42.5±9.6 | 44.9±5.6 | 13.0±4.0 |
| 48h-50µM Taurolidine | 33.9±10.2 | 44.3±5.9 | 21.8±4.6 |
| 48h-100µM Taurolidine | 25.8±1.7 | 63.2±9.8 | 11.0±11.0 |
| | | | |

| PA1 | | | |
|---|---|---|---|
| 48h-0µM Taurolidine | 29.9±1.5 | 47.7±1.0 | 22.5±0.5 |
| 48h-25µM Taurolidine | 28.4±0.5 | 46.8±0.6 | 24.7±0.9 |
| 48h-50µM Taurolidine | 23.7±2.2 | 39.5±12.5 | 36.8±12.4 |
| 48h-100µM Taurolidine | 28.4±5.6 | 44.5±23.5 | 21.2±17.9 |
| | | | |

| SKOV3 | | | |
|---|---|---|---|
| 48h-0µM Taurolidine | 46.7±1.3 | 38.8±4.1 | 13.5±3.6 |
| 48h-25µM Taurolidine | 45.8±2.7 | 41.9±4.2 | 12.3±3.1 |
| 48h-50µM Taurolidine | 30.7±9,4 | 45.5±12.4 | 30.3±10.5 |
| 48h-100µM Taurolidine | 19.9±6.1 | 54.2±8.6 | 25.9±7.8 |

Specifically, in PA-1 cells, a 48h exposure to up to 100 µM Taurolidine had little effect on cell cycle distribution. Indeed, the percentage of cells in the G₀/G₁-, S-, and G₂/M-phases were essentially unchanged despite Taurolidine exposure. Alternatively, in SKOV-3 cells, Taurolidine exposure resulted in a concentration-dependent decrease in the percentage of cells in G₀/G₁ but increased the percentage of cells in both the S-phase and G₂/M. Importantly, in both the PA-1 and SKOV-3 cell lines. Taurolidine exposure also resulted in the appearance of DNA debris in the sub-G₀G₁ region, an effect that was Taurolidine concentration-dependent (Fig. 2). Like in the SKOV-3 cell line, exposing NIH-3T3 cells to Taurolidine decreased the percentage of cells in G₀/G₁ and increased the percentage of cells in S in a concentration-dependent manner. However, unlike the human ovarian tumor cells assessed, Taurolidine exposure in NIH-3T3 cells did not significantly affect the appearance of DNA debris in the sub-G₀G₁ region (Fig. 2).

DNA cleavage into discrete fragments is a late event in the process of apoptosis. The appearance of DNA debris in the sub-G₀/G₁ region 48h after Taurolidine exposure could be a reflection of apoptosis-associated DNA fragmentation. To evaluate this possibility, studies next assessed the ability of Taurolidine to increase phosphotidylserine externalization on cell membranes, an event that occurs earlier in the apoptotic process. These studies were fluorocytometry-based and employed a florescent antibody-binding assay (Annexin-V) to assess phosphotidylserine externalization. The results of the studies (shown in Fig. 3) revealed that in both the PA-1 and SKOV-3 human ovarian tumor cell lines a 24h exposure to Taurolidine induced a significant, Taurolidine-concentration dependent, increase in Annexin-V binding of 4- and 3-fold, respectively. In contrast, in NIH-3T3 cells, Taurolidine exposure resulted in a non-significant increase (~5%) in antibody binding. These data supported the results from the cell cycle studies as well as the observation that Taurolidine exposure induced apoptosis in PA-1 and SKOV-3 cells, but not in NIH-3T3 cells. The results indicate that Taurolidine preferentially induces apoptosis (and apoptotic death) in tumor cells compared to in non-tumor cells.

To further confirm the induction of apoptosis by Taurolidine, the relationship between Taurolidine exposure and PARP cleavage was assessed. PARP is a nuclear protein that plays a key role in the recognition and repair of both single and double strand DNA breaks. In addition, a key event in the apoptotic process is the cleavage, mediated by caspase 3 and caspase 9. and consequent catalytic deactivation of this protein. To determine if Taurolidine exposure resulted in PARP cleavage in ovarian tumor cells. Western-blot analysis was carried out on whole cell extracts of PA-1, SKOV-3 and NIH-3T3 cells following a 24h exposure to either 50 or 100 µM Taurolidine. The results of this analysis, presented in the representative Western-blot contained in Fig. 4. revealed that in PA-1 and SKOV-3 cells exposure to either 50 µM or 100 µM Taurolidine resulted in PARP cleavage. In contrast, in NIH-3T3 cells, following exposure to Taurolidine there was little evidence of this proteolytic event. These data confirm that Taurolidine induces apoptosis in tumor cells but not in non-tumor cells.

Given the preferential induction of apoptotic death in tumor cells compared to normal nonneoplastic cells, Taurolidine was administered to tumor-bearing animals to further evaluate antineoplastic activity. Studies were initiated to evaluate the antineoplastic activity of taurolidine in nude mice bearing i.p. human ovarian tumor xenografts. *In vivo* studies were designed to identify the maximally tolerated dose (MTD) regimen of Taurolidine in nude female mice and to assess toxicity. Toxicity was evaluated by measuring changes in body weight, and mortality after a 3d i.p. bolus injection regimen. Daily 1ml injections delivered doses that ranged from 5mg/mouse/day - 30mg/mouse/day. The results of these studies revealed that at daily doses below 15 mg/mouse (~ 650mg/kg) Taurolidine were well-tolerated (Table 7).

Taurolidine-induced toxicity in athymic (nude) female mice was evaluated as follows, Groups of 5-10 mice were injected with Taurolidine on three consecutive days. Taurolidine doses evaluated ranged from 5-30 mg/mouse/injection and were delivered intraperitoneally in a volume of 1ml (with the exception of the 25 and 30mg doses, which, due to limited solubility, were delivered in a volume of 1.25 and 1.5ml, respectively). During the injection regimen, and daily thereafter for 30d. mice were weighed and examined. Experiments were repeated a minimum of three times and mortality and weight loss data pooled.

**Table 7**

| Taurolidine dose (mg/mouse/inj) | n | weight loss (nadir %) | mortality (%) |
|---|---|---|---|
| None (vehicle control) | 24 | -1.2 | 0 |
| 5 | 17 | -1.2 | 0 |
| 10 | 17 | -1,7 | 6% |
| 15 | 17 | -7.1 | 0 |
| 20 | 46 | -12.2 | 13% |
| 25 | 17 | -16.3 | 47% |
| 30 | 10 | -24.5 | 100% |

Maximum body weight loss as a result of this dose regimen was 7% and body weight returned to pre-injection levels within seven days after completion of the injection regimen. With regimens employing doses of 24 mg/mouse or greater, more significant toxicity was observed (Table 3). Specifically, nadir weight loss for regimens employing 20.25 or 30 mg/mouse were -12%.-16% and -25%, respectively. Additionally, these Taurolidine dose regimens resulted in 15%. 43% and 100% mortality, respectively.

Based on the toxicity studies, a 3 daily 1ml i.p. injection of Taurolidine, at a dose of 20mg/mouse, was chosen to be the MTD. Studies next evaluated the antineoplastic activity of this regimen in mice bearing i.p. human ovarian tumor xenografts derived from the SKOV-3 cell line. Mice were injected i.p. with 5 x 10⁶ SKOV-3 cells. Taurolidine therapy, employing the 3d 20mg/mouse dose regimen, was initiated up to 5d after tumor cell injection. Fourteen days following the termination of Taurolidine therapy, mice were sacrificed and all i.p. tumors removed and weighed. The results of this study, summarized in Figs. 5 and 6. revealed that, when initiated on the day of tumor cell injection. Taurolidine therapy was highly effective and inhibited tumor formation (Fig. 5), ascites development, and growth (Fig 6).

The effect of a single 3d i.p. bolus injection regimen of Taurolidine (20mg/mouse/injection, starting on the day of tumor cell injection) on the gross appearance of mice bearing i.p. xenografts of SKOV-3 human ovarian tumor cells was evaluated. Nineteen days after tumor cell injection, the mean tumor weight in control mice (no Taurolidine) was approximately 1.7gm. Additionally, control animals were found to contain up to 7ml of ascites fluid. Mean tumor weight in the taurolidine-treated group (single regimen of taurolidine) was less than 50 mgs and there was no evidence of ascites formation. A significant number of these Taurolidine-treated animals also were found to be tumor-free.

When therapy was initiated on the day of tumor cell injection, ~80% of treated mice had no evidence of disease upon sacrifice. Further, the mean tumor size in treated mice with tumors was approximately 40-fold smaller and in control (vehicle-treated) mice. Even if Taurolidine therapy was delayed for up to 3d after tumor cell injection, approximately 10 percent of mice were tumor free upon sacrifice and the mean tumor size in treated mice again was significantly smaller than in controls. The initiation of this single cycle of Taurolidine therapy 5d after tumor cell injection (i.e., in mice with established i.p. ovarian tumors) was still capable of significantly inhibiting tumor growth.
The data presented herein indicate that a class of compounds exemplified by taurolidine possesses potent antineoplastic activity by selectively inhibiting tumor cell growth and specifically induce apoptosis in tumor cells. Surprisingly, the cytotoxic IC₅₀ of Taurolidine was found to be in the 10-50 µM range, approximately 100-fold lower that that required for its antibiotic effects. This difference in effective concentrations, combined with Taurolidine's observed low clinical toxicities indicates that this class of compounds is useful as a safe, clinically well-tolerated antineoplastic.

The data revealed that exposure to Taurolidine effectively inhibited the proliferation and viability of all tumor cell lines evaluated in a broad panel of solid tumor cell lines. Taurolidine induced apoptosis in neoplastic cells, indicating that its mechanism of action is not simply an inhibition of cell surface adhesion components or processes. Results of studies carried out in non-adherent cancer cell models support the above findings and reveal that as little as a 90min exposure to Taurolidine induces apoptosis in the HL-60 human promyelocytic cell line. Exposure to Taurolidine results in the activation of caspases 3, 8 and 9. a disruption of mitochondrial membrane integrity accompanied by cytochrome-C efflux from these organelles, and the cleavage of PARP protein.

Human leukemia HL-60 cells, which were genetically-engineered to resist apoptotic induction, were induced to apoptosis independently (downstream) of the bc1-2/bax (anti-death gene) point in the signal transduction cascade. Surprising, in Bcl2-over expressing HL-60 cells, Taurolidine exposure was found to be capable of inducing apoptosis, but with a delayed onset. These data indicate that an active Taurolidine breakdown product is capable of reacting with membrane components to affect intracellular signaling processes and initiate the apoptosis process.

The ability of Taurolidine to induce apoptosis was found to be specific for tumor cells. This observation was confirmed using normal (non-tumor) primary cells derived from animals, which are known to be free of tumors. The cytotoxic and apoptotic activity of Taurolidine in normal murine bone marrow cultures as well as in activated human T-cell cultures was evaluated. In both normal cell models. Taurolidine was not cytotoxic in the high µM range and did not produce cellular changes consistent with the induction of apoptosis. In normal murine bone marrow, concentrations in the mM range were required to inhibit cell proliferation. These findings indicate that Taurolidine (or one of its metabolites) access a tumor-cell specific target capable of inducing tumor cell apoptosis.

### Example 2: Clinical Use

Taurolidine was administered by i.p. lavage immediately following surgery for removal of recurrent ovarian tumors. For patients with glioblastoma. Taurolidine was administered systemically. To date. Taurolidine has been well tolerated in these patients.

Four patients, which were diagnosed with advanced recurrent glioblastoma multiforma, were treated with taurolidine. The prognosis for this group of patients was determined to be approximately 8 weeks of survival. Each patient received at least one 5 week regimen in which 20 g of taurolidine was infused intravenously into the arm over a period of 6 hours twice a week. In 3 out of the 4 patients treated, the tumor mass decreased or stayed the same; and in one case, a slight increase was seen. At 14 weeks after the initiation of therapy, each of the patients remains alive, having exceeded the 8 week prognosis. A beneficial clinical effect was achieved in these brain tumor patients with systemic administration of taurolidine, indicating that taurolidine, or a metabolite of taurolidine, successfully crossed the blood-brain barrier to gain access to the tumor in the brain.

These data indicate that taurolidine and derivatives or metabolites thereof are useful to inhibit or halt tumor growth and to extend the life expectancy of tumor patients.

Other embodiments are within the following claims.

## Claims

1. A compound for use in inducing apoptosis in cancer cells in a mammal, wherein said compound is taurolidine or taurultam and wherein said cancer is selected from colon cancer, lung cancer and prostate cancer.

2. A compound as claimed in claim 1 for the use according to claim 1, wherein said cancer cells express a gene encoding multiple drug resistance.

3. A compound as claimed in claim 1 or 2 for the use according to claim 1, for administration to directly contact said cancer cells.

4. A compound as claimed in claim 3 for the use according to claim 1, formulated as a drug-loaded matrix for implantation in direct contact with or adjacent to said cancer cells.

5. A compound as claimed in claim 1 or 2 for the use according to claim 1, formulated for intraperitoneal administration.

6. A compound as claimed in any of claims 1-3 for the use according to claim 1, for administration prior to, simultaneously with, or subsequent to the administration of a second chemo-therapeutic agent which is selected from an antimetabolite, a purine analogue, a pyrimidine analogue, an alkylating agent, an intercalating agent, a crosslinking agent and an antibiotic.

7. Taurolidine for use in the treatment of advanced recurrent glioblastoma multiforme by systemic administration.

## Patentansprüche

1. Verbindung zur Verwendung bei der Induzierung von Apoptose in Krebszellen in einem Säugetier, wobei die Verbindung Taurolidin oder Taurultam ist und wobei der Krebs aus Darmkrebs, Lungenkrebs und Prostatakrebs ausgewählt wird.

2. Verbindung nach Anspruch 1 für die Anwendung nach Anspruch 1, wobei die Krebszellen ein Gen exprimieren, das eine Mehrfachresistenz codiert.

3. Verbindung nach Anspruch 1 oder 2 für die Anwendung nach Anspruch 1 zur Verabreichung mit direktem Kontakt zu den Krebszellen.

4. Verbindung nach Anspruch 3 zur Anwendung nach Anspruch 1, formuliert als medikamentenbeladene Matrix zur Implantation in direktem Kontakt mit den Krebszellen oder neben denselben.

5. Verbindung nach Anspruch 1 oder 2 zur Anwendung nach Anspruch 1, formuliert zur intraperitonealen Verabreichung.

6. Verbindung nach einem der Ansprüche 1-3 zur Anwendung nach Anspruch 1 zur Verabreichung vor, gleichzeitig mit oder nach der Verabreichung eines zweiten Chemotherapeutikums, das aus einem Antimetaboliten, einem Purinanalogon, einem Pyrimidinanalogon, einem Alkylierungsmittel, einem Intercalationsmittel, einem Vernetzungsmittel und einem Antibiotikum ausgewählt wird.

7. Taurolidin zur Anwendung bei der Behandlung von fortgeschrittenem Glioblastoma multiforme durch systemische Verabreichung.

## Revendications

1. Composé destiné à être utilisé pour induire l'apoptose dans les cellules cancéreuses chez un mammifère, dans lequel ledit composé est la taurolidine ou le taurultam et dans lequel ledit cancer est sélectionné parmi un cancer du côlon, un cancer du poumon et un cancer de la prostate.

2. Composé selon la revendication 1 destiné à être utilisé selon la revendication 1, dans lequel lesdites cellules cancéreuses expriment un gène codant pour la résistance multi-médicamenteuse.

3. Composé selon la revendication 1 ou 2 destiné à être utilisé selon la revendication 1, pour une administration de manière à venir en contact direct avec lesdites cellules cancéreuses.

4. Composé selon la revendication 3 destiné à être utilisé selon la revendication 1, formulé comme une matrice chargée de médicament pour une implantation en contact direct avec lesdites cellules cancéreuses ou adjacente à celles-ci.

5. Composé selon la revendication 1 ou 2 destiné à être utilisé selon la revendication 1, formulé pour une injection intrapéritonéale.

6. Composé selon l'une quelconque des revendications 1 à 3 destiné à être utilisé selon la revendication 1, pour une administration avant, en même temps que, ou après, l'administration d'un second agent chimiothérapeutique sélectionné parmi un antimétabolite, un analogue de la purine, un analogue de la pyrimidine, un agent d'alkylation, un agent d'intercalation, un agent de réticulation et un antibiotique.

7. Taurolidine destinée à être utilisée dans le traitement d'un glioblastome multiforme récurrent à un stade avancé par administration systémique.
